# EUROPEAN PATENT APPLICATION

(11) **EP 3 932 307 A1**
(43) Date of publication of application: **05.01.2022**
(21) Application number: 20762478.4
(22) Date of filing: 14.01.2020
(51) Int. Cl.: A61B 5/11, A41D 1/00, A41D 13/00, A61B 5/00, A61B 5/113, G01D 5/353

(54) **OPTICAL FIBER SENSING SYSTEM, STATE DETECTION DEVICE, STATE DETECTION METHOD, AND NON-TRANSITORY COMPUTER-READABLE MEDIUM**

(30) Priority: 26.02.2019 JP 2019033252
(71) Applicant: NEC Corporation, 108-8001 Tokyo (JP)
(72) Inventor: YODA Yukihide, Tokyo 108-8001 (JP); ISHII Satoru, Tokyo 108-8001 (JP); AONO Yoshiaki, Tokyo 108-8001 (JP)
(74) Representative: Reeve, Nicholas Edward
(86) International application number: PCT/JP2020/000883
(87) International publication number: WO 2020/174901

(57) **Abstract**

An optical fiber sensing system according to the present disclosure includes clothing (10) including an optical fiber (20), an optical fiber sensing unit (31) configured to receive an optical signal from the optical fiber (20), and acquire, based on the optical signal, a plurality of parameters each having a pattern according to a state of a person wearing the clothing (10), and a state detection unit (32) configured to detect a state of a person wearing the clothing (10), based on a pattern contained in each of the plurality of parameters.

## Description

### Technical Field

The present disclosure relates to an optical fiber sensing system, a state detection device, a state detection method, and a non-transitory computer-readable medium.

### Background Art

In recent years, as a technique for detecting a state of a monitoring target (mainly a person) in detail, pattern sensing using a dynamic pattern of optical fiber sensing has been expected as a future technique.

Vibration data that can be detected by optical fiber sensing have unique patterns according to a state of a monitoring target, and analyzing a dynamic change of each of the patterns enables detection of the state of the monitoring target.

Moreover, recently, applying pattern sensing of optical fiber sensing to clothing has also been suggested (e.g., Patent Literature 1). A technique described in Patent Literature 1 fixes or mixes an optical fiber into clothing. When vibration occurred by movement or a biological activity of a person is given to an optical fiber, a polarization fluctuation occurs in an optical signal propagating in the optical fiber. The technique described in Patent Literature 1 detects a biological activity and mobility/immobility of a person by detecting the polarization fluctuation.

### Citation List

### Patent Literature

[Patent Literature 1] Japanese Unexamined Patent Application Publication No. 2007-061306

### Summary of Invention

### Technical Problem

However, the technique described in Patent Literature 1 uses only vibration data to monitor a biological activity and the like of a person. Thus, detecting a state of a person resulting from a cause other than vibration is difficult.

On the other hand, while optical fiber sensing is capable of detecting not only vibration but also sound and temperature, high-level state detection using the characteristics is also expected.

Accordingly, an object of the present disclosure is to provide an optical fiber sensing system, a state detection device, a state detection method, and a non-transitory computer-readable medium that solve the above-described problem, and can detect a state of a person at a higher level and flexibly.

### Solution to Problem

An optical fiber sensing system according to one aspect includes:
clothing including an optical fiber;
an optical fiber sensing unit configured to receive an optical signal from the optical fiber, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

A state detection device according to one aspect includes:
an optical fiber sensing unit configured to receive an optical signal from an optical fiber included in clothing, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

A state detection method according to one aspect is
a state detection method by a state detection device, including:
receiving an optical signal from an optical fiber included in clothing, and acquiring, based on the optical signal, a plurality of parameters each having a pattern according to a state of a person wearing the clothing; and
detecting a state of a person wearing the clothing, based on a pattern contained in each of the plurality of parameters.

A non-transitory computer-readable medium according to one aspect is
a non-transitory computer-readable medium storing a program that causes a computer to execute:
a procedure of receiving an optical signal from an optical fiber included in clothing, and acquiring, based on the optical signal, a plurality of parameters each having a pattern according to a state of a person wearing the clothing; and
a procedure of detecting a state of a person wearing the clothing, based on a pattern contained in each of the plurality of parameters.

### Advantageous Effects of Invention

According to the above-described aspects, an advantageous effect can be acquired in which an optical fiber sensing system, a state detection device, a state detection method, and a non-transitory computer-readable medium that can detect a state of a person at a higher level and flexibly can be provided.

### Brief Description of the Drawings

Fig. 1 is a diagram illustrating a configuration example of an optical fiber sensing system according to a first example embodiment.
Fig. 2 is a diagram illustrating another example of clothing according to the first example embodiment.
Fig. 3 is a diagram illustrating an example of a connection aspect between an optical fiber and an optical fiber sensing unit according to the first example embodiment.
Fig. 4 is a block diagram illustrating a configuration example of the optical fiber sensing unit in a case of the connection aspect in Fig. 3.
Fig. 5 is a block diagram illustrating another configuration example of the optical fiber sensing unit in a case of the connection aspect in Fig. 3.
Fig. 6 is a diagram illustrating another example of a connection aspect between the optical fiber and the optical fiber sensing unit according to the first example embodiment.
Fig. 7 is a block diagram illustrating a configuration example of the optical fiber sensing unit in a case of the connection aspect in Fig. 6.
Fig. 8 is a diagram illustrating an example of a part information table according to the first example embodiment.
Fig. 9 is a diagram illustrating an example of vibration data acquired by the optical fiber sensing unit according to the first example embodiment.
Fig. 10 is a diagram illustrating another example of vibration data acquired by the optical fiber sensing unit according to the first example embodiment.
Fig. 11 is a flow diagram illustrating an example of machine learning executed by a state detection unit according to the first example embodiment.
Fig. 12 is a diagram illustrating an example of state information according to the first example embodiment.
Fig. 13 is a diagram illustrating an example of a method of detecting a sign of an abnormal state of a person wearing clothing, in the state detection unit according to the first example embodiment.
Fig. 14 is a diagram illustrating an example of a manufacturing method of clothing according to the first example embodiment.
Fig. 15 is a diagram illustrating an example of a breaking prevention measure for an optical fiber according to the first example embodiment.
Fig. 16 is a diagram illustrating another example of a breaking prevention measure for an optical fiber according to the first example embodiment.
Fig. 17 is a diagram illustrating an example of high-density arrangement of an optical fiber according to the first example embodiment.
Fig. 18 is a block diagram illustrating an example of a hardware configuration of a computer that achieves a state detection device according to the first example embodiment.
Fig. 19 is a flow diagram illustrating an example of an operation flow of the optical fiber sensing system according to the first example embodiment.
Fig. 20 is a diagram illustrating a configuration example of an optical fiber sensing system according to a second example embodiment.
Fig. 21 is a diagram illustrating an example of a connection aspect between an optical fiber sensing unit according to the second example embodiment and a user terminal.
Fig. 22 is a block diagram illustrating a configuration example of the optical fiber sensing system in a case of the connection aspect in Fig. 21.
Fig. 23 is a diagram illustrating another example of a connection aspect between the optical fiber sensing unit according to the second example embodiment and the user terminal.
Fig. 24 is a block diagram illustrating a configuration example of the optical fiber sensing unit in a case of the connection aspect in Fig. 23.
Fig. 25 is a diagram illustrating an example of a GUI screen indicating a state detection result being generated by a state detection unit according to the second example embodiment and transmitted to a user terminal.
Fig. 26 is a diagram illustrating a configuration example of a chair including an optical fiber according to another example embodiment.
Fig. 27 is a diagram illustrating an example of a part information table used in the another example embodiment in Fig. 26.
Fig. 28 is a diagram illustrating a configuration example of a bed including an optical fiber according to another example embodiment.
Fig. 29 is a diagram illustrating an example of a part information table used in the another example embodiment in Fig. 28.

### Description of Embodiments

Hereinafter, example embodiments of the present disclosure are described with reference to the drawings.

### <First Example Embodiment>

### <Configuration according to First Example Embodiment

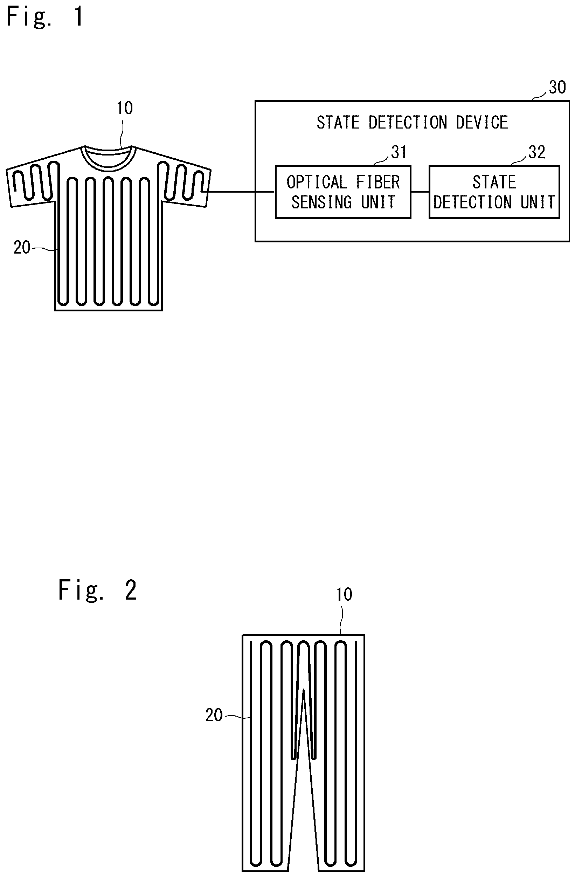

First, a configuration of an optical fiber sensing system according to a first example embodiment is described with reference to Fig. 1.

As illustrated in Fig. 1, the optical fiber sensing system according to the first example embodiment includes clothing 10 including an optical fiber 20, and a state detection device 30. Moreover, the state detection device 30 includes an optical fiber sensing unit 31 and a state detection unit 32. Note that, although the state detection device 30 includes the optical fiber sensing unit 31 and the state detection unit 32 in the example of Fig. 1, the present disclosure is not limited thereto. The optical fiber sensing unit 31 and the state detection unit 32 may be each achieved by a separate device, and have a mutually communicable configuration.

The clothing 10 includes the optical fiber 20. The optical fiber 20 may be sewn into a cloth part of the clothing 10, or may be fixed to and laid on the clothing 10. Moreover, the optical fiber 20 may be included in the clothing 10 in an aspect of an optical fiber cable configured by covering the optical fiber 20. Moreover, although the clothing 10 is clothing for an upper body such as a shirt in the example of Fig. 1, the present disclosure is not limited thereto. As illustrated in Fig. 2, the clothing 10 may be clothing for a lower body such as pants.

The optical fiber sensing unit 31 is connected to the optical fiber 20, and brings pulsed light into the optical fiber 20. Moreover, the optical fiber sensing unit 31 receives, from the optical fiber 20, backward reflected light occurred at each transmission distance due to transmission of pulsed light through the optical fiber 20.

Herein, when a person wears the clothing 10, vibration of a bodily organ such as a heart of the person wearing the clothing 10, for example, is transmitted to the optical fiber 20, and the vibration is superimposed on backward reflected light transmitted by the optical fiber 20. Moreover, when a person wearing the clothing 10 falls or collides with something, vibration at this point is also transmitted to the optical fiber 20, and the vibration is also superimposed on the backward reflected light transmitted by the optical fiber 20. Thus, the optical fiber sensing unit 31 is capable of detecting vibration occurred in a person wearing the clothing 10, based on backward scattered light received from the optical fiber 20. Moreover, the optical fiber sensing unit 31 is also capable of detecting, based on a time from bringing of pulsed light into the optical fiber 20 to reception of vibration-superimposed backward scattered light from the optical fiber 20, an occurrence position (a distance from the optical fiber sensing unit 31) where the backward scattered light occurs.

For example, the optical fiber sensing unit 31 is capable of detecting, by detecting, with a distributed vibration sensor, the backward scattered light received from the optical fiber 20, vibration occurred in a person wearing the clothing 10 and an occurrence position of backward scattered light on which the vibration is superimposed, and acquiring vibration data of the detected vibration.

Herein, as described above, vibration detected by the optical fiber sensing unit 31 is vibration of a bodily organ such as a heart of a person wearing the clothing 10, vibration occurred at falling or collision of the person wearing the clothing 10, or the like. Thus, vibration data of vibration detected by the optical fiber sensing unit 31 have a unique pattern differing in transition of fluctuation in strength and weakness of the vibration, a vibration position, the number of vibrations, and the like, according to a state of a person wearing the clothing 10 (a physical condition or an action of a person).

Thus, the state detection unit 32 becomes capable of identifying a state of a person wearing the clothing 10, by analyzing a dynamic change of a unique pattern contained in the vibration data acquired by the optical fiber sensing unit 31.

Moreover, sound (e.g., heart sound, tracheal sound, bronchial sound, or the like) and temperature (e.g., a bodily temperature or the like) occurred in a person wearing the clothing 10 are also superimposed on backward scattered light transmitted by the optical fiber 20. Thus, the optical fiber sensing unit 31 is capable of detecting sound and temperature occurred in a person wearing the clothing 10, based on the backward scattered light received from the optical fiber 20.

For example, the optical fiber sensing unit 31 is capable of detecting, by detecting the backward scattered light received from the optical fiber 20 with each of a distributed acoustic sensor and a distributed temperature sensor, sound and temperature occurred in a person wearing the clothing 10, and acquiring acoustic data and temperature data of the detected sound and temperature.

Herein, sound detected by the optical fiber sensing unit 31 is heart sound and the like occurred in a person wearing the clothing 10, as described above. Moreover, temperature detected by the optical fiber sensing unit 31 is a bodily temperature and the like of a person wearing the clothing 10, as described above. Thus, acoustic data of sound and temperature data of temperature detected by the optical fiber sensing unit 31 also each have a unique pattern according to a state of a person wearing the clothing 10.

Thus, in the present first example embodiment, the state detection unit 32 performs detection of a state of a person wearing the clothing 10, based on a pattern contained in each of a plurality of parameters of vibration, sound, and temperature according to a state of a person wearing the clothing 10. Consequently, a state of a person wearing the clothing 10 can be detected at a higher level and flexibly.

Hereinafter, the optical fiber sensing system according to the present first example embodiment is described in detail.

### <Connection Aspect between Optical Fiber 20 and Optical Fiber Sensing Unit 31>

First, a connection aspect between the optical fiber 20 and the optical fiber sensing unit 31 is described.

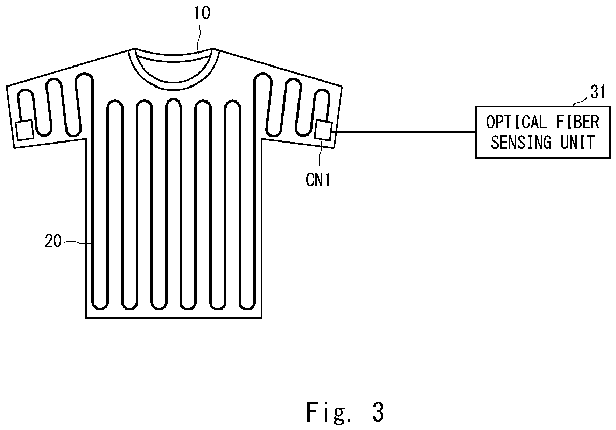

For example, as illustrated in Fig. 3, on the clothing 10 side, a connector CN1 may be attached to an end of the optical fiber 20, and the optical fiber 20 may be connected to the optical fiber sensing unit 31 by use of the connector CN1.

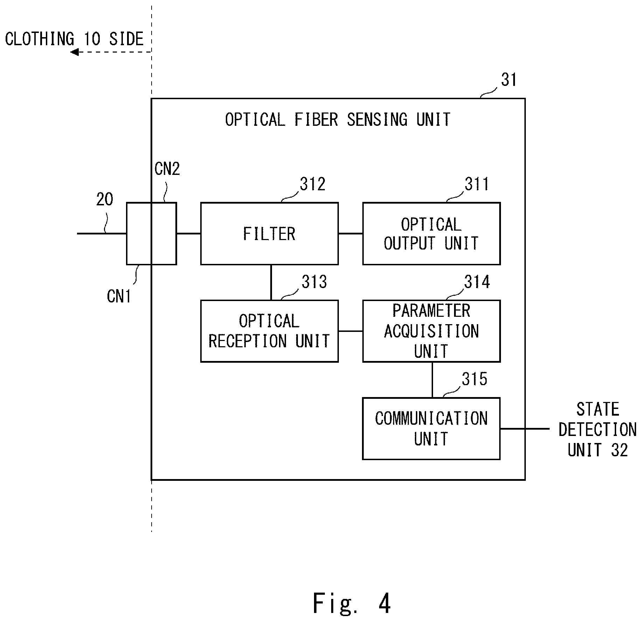

A configuration example of the optical fiber sensing unit 31 in a case of the connection aspect in Fig. 3 is illustrated in Fig. 4.

The optical fiber sensing unit 31 illustrated in Fig. 4 includes a connector CN2, an optical output unit 311, a filter 312, an optical reception unit 313, a parameter acquisition unit 314, and a communication unit 315.

The connector CN2 is connected to the connector CN1 on the clothing 10 side.

The optical output unit 311 brings pulsed light into the optical fiber 20 via the filter 312 and the connectors CN1 and CN2.

The optical reception unit 313 receives backward reflected light from the optical fiber 20 via the connectors CN1 and CN2 and the filter 312.

The parameter acquisition unit 314 acquires, based on backward scattered light received by the optical reception unit 313, a plurality of parameters (e.g., including at least two parameters among three parameters of vibration data, acoustic data, and temperature data) each having a pattern according to a state of a person wearing the clothing 10.

The communication unit 315 outputs, to the state detection unit 32, the plurality of parameters acquired by the parameter acquisition unit 314.

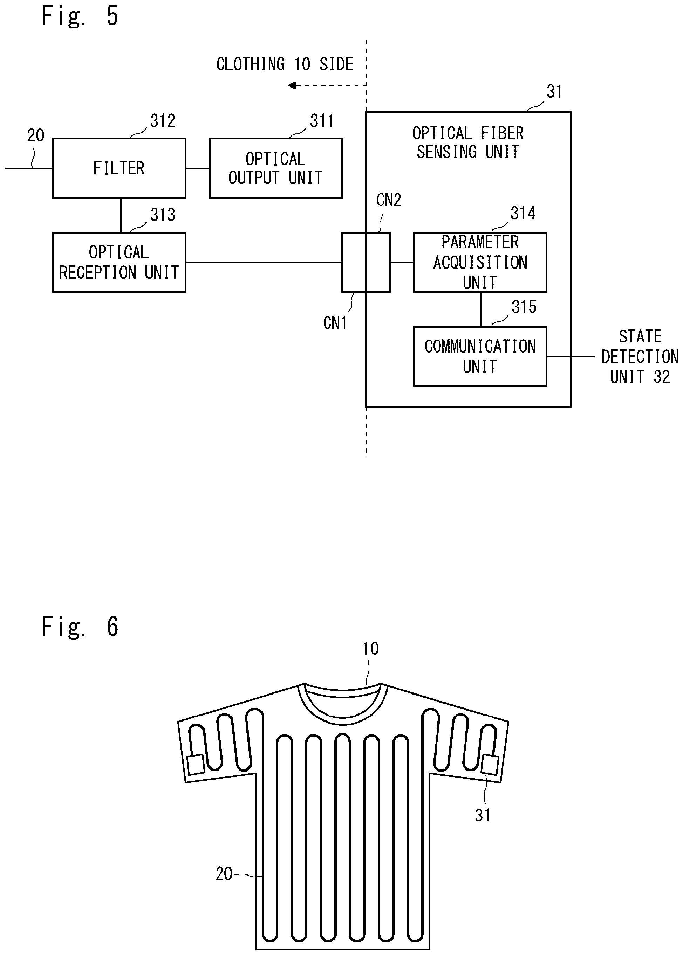

Moreover, as illustrated in Fig. 5, among components of the optical fiber sensing unit 31 illustrated in Fig. 4, the optical output unit 311, the filter 312, and the optical reception unit 313 may be provided on the clothing 10 side. In this case, the parameter acquisition unit 314 inputs, via the connectors CN1 and CN2, the backward reflected light received by the optical reception unit 313.

Moreover, as illustrated in Fig. 6, on the clothing 10 side, the optical fiber sensing unit 31 may be attached to an end of the optical fiber 20, and the optical fiber 20 may be directly connected to the optical fiber sensing unit 31.

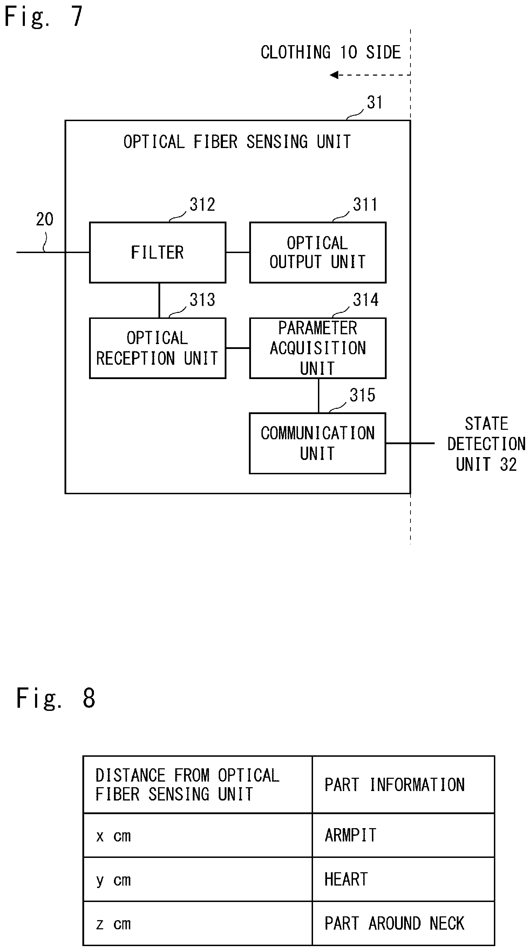

A configuration example of the optical fiber sensing unit 31 in a case of the connection aspect in Fig. 6 is illustrated in Fig. 7.

The optical fiber sensing unit 31 illustrated in Fig. 7 is equivalent to a configuration in which the connector CN2 is removed from the configuration illustrated in Fig. 4, and provided on the clothing 10 side.

### <Identification Method of Bodily Part>

As described above, the optical fiber sensing unit 31 is capable of detecting, based on a time from bringing of pulsed light into the optical fiber 20 to reception of backward scattered light from the optical fiber 20, an occurrence position (a distance from the optical fiber sensing unit 31) of the backward scattered light.

However, in order to detect a state of a person, it is necessary to specify which part of a body an occurrence position of backward scattered light is in.

Thus, an example of a method of specifying, in the state detection unit 32, which part of a body an occurrence position of backward scattered light is in is described.

### (A1) Method A1

In the present method A1, the state detection unit 32 previously holds a part information table associating a distance from the optical fiber sensing unit 31 with part information indicating a part of a body being located at the distance, and specifies the part of the body by use of the part information table. Fig. 8 illustrates an example of a part information table when the clothing 10 is clothing for an upper body. It is preferable that the part information table is a table generated based on a size of a standard body of a person wearing the clothing 10 by determining the size of the standard body from a size or type of the clothing 10.

### (A2) Method A2:

In the present method A2, the state detection unit 32 generates, by manual learning, the above-described part information table as illustrated in Fig. 8, and specifies a part of a body by use of the part information table. Generation of the part information table is performed, for example, as follows.

### Step S11:

On a user terminal (e.g., a smart device, a wearable device, a personal computer, and the like) owned by a person wearing the clothing 10, the state detection unit 32 instructs the person wearing the clothing 10 to tap, at regular intervals, a specific part of a body to be a monitoring target by hand. Then, the state detection unit 32 acquires vibration data at this point from the optical fiber sensing unit 31.

### Step S12:

The state detection unit 32 recognizes, on the vibration data, a place where vibration occurs at regular intervals, as a specific part to be a monitoring target. Then, the state detection unit 32 records a distance of the place from the optical fiber sensing unit 31 and the specific part, in association with each other.

The state detection unit 32 generates the above-described part information table as illustrated in Fig. 8, by repeatedly performing the above-described steps S11 and S12 for each of a plurality of specific parts to be monitoring targets.

Note that, although the state detection unit 32 instructs a person wearing the clothing 10 to tap, at regular intervals, a specific part by hand in step S11, an instruction content is not limited thereto. The state detection unit 32 may instruct a person to make such movement that a specific part to be a monitoring target vibrates, and the instruction may be, for example, such an instruction as to move an arm, raise an arm, turn an arm, or bend an elbow.

### <Detection Method of State of Person>

The state detection unit 32 performs detection of a state of a person wearing the clothing 10, based on a pattern according to the state of the person wearing the clothing 10, contained in each of a plurality of parameters (e.g., including at least two parameters among three parameters of vibration data, acoustic data, and temperature data). Hereinafter, an example of detecting a state of a person wearing the clothing 10 is described.

### (B1) When vibration data are used

Herein, an example of detecting a state of a person, based on a pattern contained in vibration data of vibration occurred in the person wearing the clothing 10, is described first.

Concentrated monitoring needs to be performed on an important bodily organ such as a heart. Thus, an example of a method of performing detection of a state of a heart of a person, based on a pattern contained in vibration data of vibration occurred near the heart of the person wearing the clothing 10, is described below as one example.

### (B11) Method B11

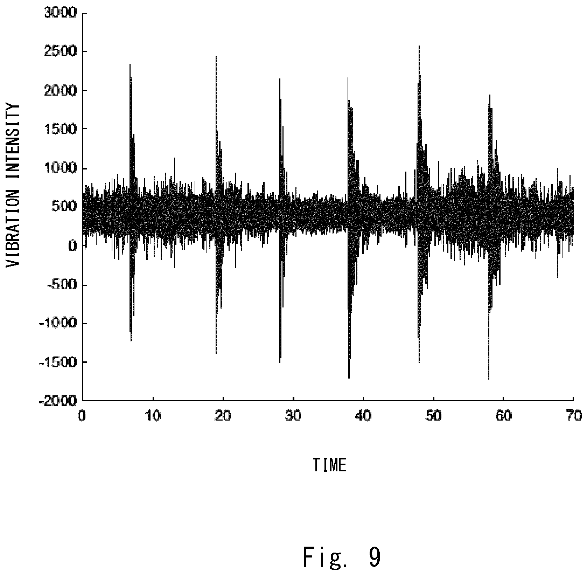

First, the present method B11 is described with reference to Fig. 9. Fig. 9 illustrates vibration data (time on a horizontal axis, and vibration intensity on a vertical axis) of vibration occurred near a heart of a person wearing the clothing 10.

In the vibration data illustrated in Fig. 9, an interval of vibration, and an attenuation pattern when vibration attenuates differ between a normal time and an abnormal time of the heart.

Thus, the state detection unit 32 previously holds vibration data (vibration data similar to those in Fig. 9) at a normal time of a heart of a person wearing the clothing 10.

When detecting a state of a heart of a person wearing the clothing 10, the state detection unit 32 acquires, from the optical fiber sensing unit 31, vibration data (vibration data similar to those in Fig. 9) of monitoring vibration occurred near the heart of the person. Then, the state detection unit 32 determines whether the heart is in an abnormal state, by whether an interval and an attenuation pattern of vibration in the monitored vibration data differ from an interval and an attenuation pattern of vibration in vibration data at a normal time.

### (B12) Method B12:

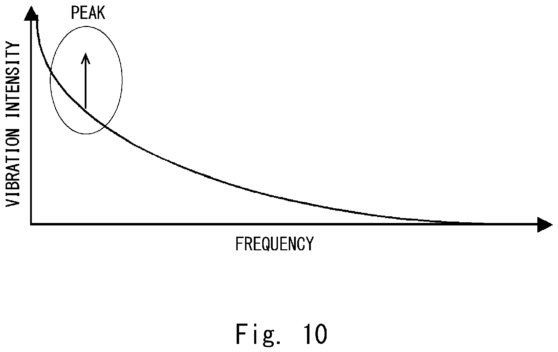

Further, the present method B12 is described with reference to Fig. 10. Fig. 10 illustrates vibration data (frequency on a horizontal axis, and vibration intensity on a vertical axis) after vibration data (vibration data similar to those in Fig. 9) of vibration occurred near a heart of a person wearing the clothing 10 are subjected to fast Fourier transform (FFT).

In the vibration data illustrated in Fig. 10, a peak of vibration intensity occurs. A frequency at which the peak occurs differs between a normal time and an abnormal time of a heart.

Thus, the state detection unit 32 previously holds vibration data (vibration data similar to those in Fig. 10) at a normal time of a heart of a person wearing the clothing 10.

When detecting a state of a heart of a person wearing the clothing 10, the state detection unit 32 acquires, from the optical fiber sensing unit 31, vibration data (vibration data similar to those in Fig. 10) of monitoring vibration occurred near the heart of the person. Then, the state detection unit 32 determines whether the heart is in an abnormal state, by whether a frequency at which a peak occurs in the monitored vibration data differs from a frequency at which a peak occurs in the vibration data at a normal time.

### (B13) Method B13

In the present method B13, a pattern according to a state of a heart of a person is subjected to machine learning (e.g., deep learning or the like) as a pattern contained in vibration data of vibration occurred near the heart of the person, and the state of the heart is determined by use of a learning result (an initial training model) of the machine learning.

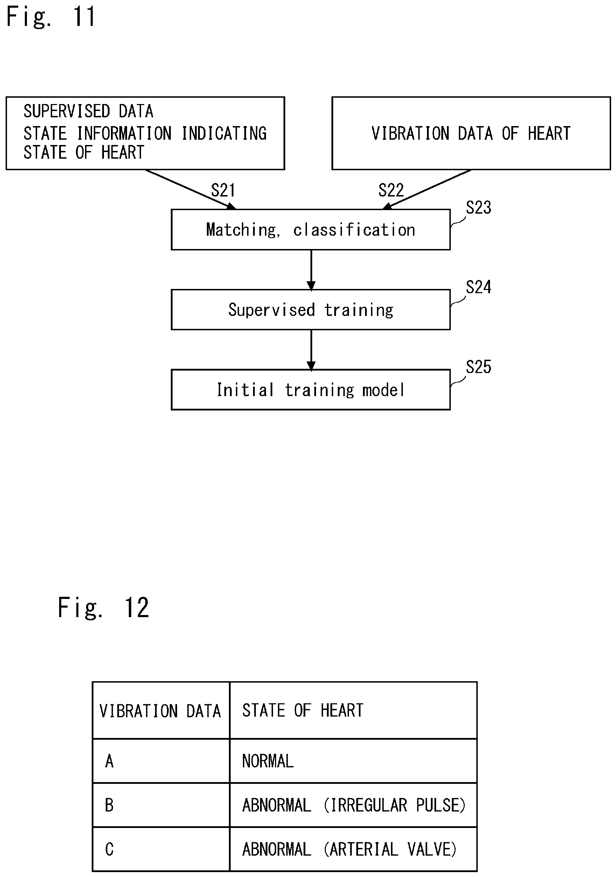

A method of machine learning in the present method B13 is described with reference to Fig. 11.

As illustrated in Fig. 11, the state detection unit 32 inputs supervised data being state information indicating a state of a heart of a person, and vibration data of vibration occurred near the heart in the state (steps S21 and S22). An example of state information to be supervised data is illustrated in Fig. 12. Fig. 12 is an example of state information when three pieces of vibration data A, B, and C are learned. Vibration data are, for example, vibration data as illustrated in Figs. 9 and 10.

Further, the state detection unit 32 performs matching and classification of both pieces of the data (step S23), and performs supervised training (step S24). Consequently, an initial training model is acquired (step S25). The initial training model becomes a model from which a state of a heart is output when monitored vibration data of the heart are input.

When detecting a state of a heart of a person wearing the clothing 10, the state detection unit 32 acquires, from the optical fiber sensing unit 31, vibration data (vibration data similar to those in Fig. 9 or 10) of monitoring vibration occurred near the heart of the person, and inputs the vibration data to the initial training model. Thus, the state detection unit 32 acquires a state of the heart of the person as an output result of the initial training model.

In the present method B13, a pattern according to a state of a heart of a person is subjected to machine learning as a pattern contained in vibration data of vibration occurred near the heart of the person, and the state of the heart of the person is determined by use of a learning result of the machine learning.

Extracting a feature for detecting a state of a heart from vibration data may be difficult for an analysis by a human. In the present method B13, a state of a heart can be detected with a high degree of accuracy by building a training model from a large amount of vibration data, even when a state analysis by a human is difficult.

In machine learning in the present method B13, a training model may be generated, based on two or more pieces of supervised data in an initial state. Moreover, the training model may be caused to newly learn vibration data of a heart newly acquired by the optical fiber sensing unit 31. In this instance, a detailed condition for detecting a state of a heart may be adjusted from a new training model.

Note that, although the above-described methods B11 to B13 have been each described above as a method of detecting a state of a heart, the above-described methods B11 to B13 are also applicable to a bodily organ other than a heart.

Moreover, vibration of a bodily organ of a person wearing the clothing 10 also depends on status such as motion of the person. However, since vibration and temperature of the whole body of the person are also monitored in the present first example embodiment, determination of a motion state of the whole body during motion/a break/a meal and the like is possible. This enables high-level state detection by performing monitoring of vibration occurred in each bodily organ, in conformity to a present motion state of the whole body.

As described above, the state detection unit 32 is capable of detecting an abnormal state (e.g., abnormality of a heart, such as an irregular pulse or an arterial valve) of a bodily organ by monitoring a pattern contained in vibration data of vibration occurred in the bodily organ of a person wearing the clothing 10.

Moreover, the state detection unit 32 is also capable of detecting a heartbeat, a pulse, blood pressure, and the like as a state of a person, by monitoring a pattern contained in vibration data of vibration occurred in the person wearing the clothing 10.

Moreover, when strong vibration is given to the optical fiber 20 from outside by falling or collision of a person wearing the clothing 10, the state detection unit 32 is also capable of detecting a state (e.g., falling or collision) to be a cause of the vibration, by monitoring a pattern contained in vibration data of the vibration.

### (B2) When acoustic data are used

Further, an example of detecting a state of a person, based on a pattern contained in acoustic data of sound occurred in the person wearing the clothing 10, is described.

For example, the state detection unit 32 is capable of detecting various abnormal states (e.g., asthma , pneumonia, pleural effusion, and the like) of a person, by monitoring a pattern contained in acoustic data of breathing sound occurred in a trachea or a bronchus of the person wearing the clothing 10.

Moreover, the state detection unit 32 is also capable of detecting various abnormal states of a person, by monitoring a pattern contained in acoustic data of abdominal sound occurred in an abdomen of the person wearing the clothing 10.

Note that, for example, a method similar to the above-described methods B11 to B13 for vibration data may be used as a method of detecting an abnormal state of a person wearing the clothing 10 by use of acoustic data.

In other words, the state detection unit 32 may previously hold acoustic data at a normal time, and determine whether a person is in an abnormal state, by whether a pattern contained in monitored acoustic data differs from a pattern contained in the acoustic data at a normal time.

Alternatively, the state detection unit 32 may subject a pattern according to a state of a person to machine learning (e.g., deep learning or the like) as a pattern contained in acoustic data of sound occurred in the person wearing the clothing 10, and determine a state of the person by use of a learning result (an initial training model) of the machine learning.

### (B3) When temperature data are used

Further, an example of detecting a state of a person, based on a pattern contained in temperature data of temperature occurred in the person wearing the clothing 10, is described.

For example, the state detection unit 32 is capable of detecting various abnormal states (e.g., a fever, a swelling in a body, and the like) of a person, by monitoring a pattern contained in temperature data of a bodily temperature of the person wearing the clothing 10.

Note that, for example, a method similar to the above-described methods B11 to B13 for vibration data may be used as a method of detecting an abnormal state of a person wearing the clothing 10 by use of temperature data.

In other words, the state detection unit 32 may previously hold temperature data at a normal time, and determine whether a person is in an abnormal state, by whether a pattern contained in monitored temperature data differs from a pattern contained in the temperature data at a normal time.

Alternatively, the state detection unit 32 may subject a pattern according to a state of a person to machine learning (e.g., deep learning or the like) as a pattern contained in temperature data of temperature occurred in the person wearing the clothing 10, and determine a state of the person by use of a learning result (an initial training model) of the machine learning.

As described above, the state detection unit 32 is capable of detecting various states of a person only by individually monitoring patterns contained in vibration data, acoustic data, and temperature data occurred in the person wearing the clothing 10.

In the present first example embodiment, the state detection unit 32 monitors, for example, a pattern contained in at least two parameters among three parameters of vibration data, acoustic data, and temperature data, and detects a state of a person wearing the clothing 10. Thus, a plurality of states (e.g., abnormality of a heartbeat, abnormality of a breathing sound, abnormality of a bodily temperature, and the like) of the person wearing the clothing 10 can be detected, and this enables detecting a state of the person at a higher level and flexibly.

### <Detection of sign of abnormal state of person>

The state detection unit 32 may detect a sign of an abnormal state of a person, based on a state change of the person wearing the clothing 10 with time.

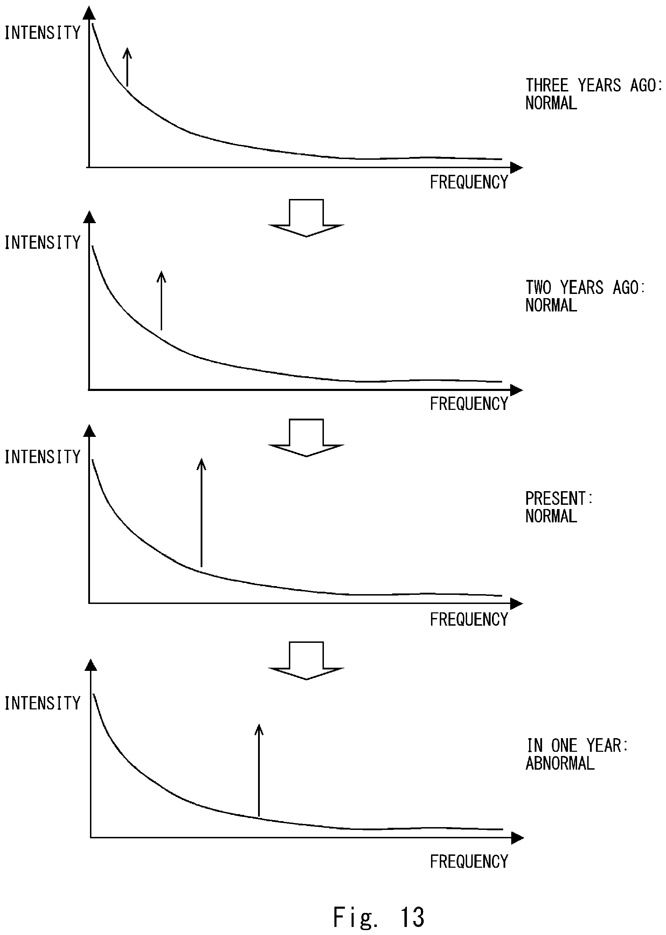

Herein, an example of a method of detecting, in the state detection unit 32, a sign of an abnormal state of a person wearing the clothing 10 is described with reference to Fig. 13. Fig. 13 illustrates vibration data similar to those in Fig. 10 in a time-series layout.

As illustrated in Fig. 13, the state detection unit 32 predicts vibration data of a person in one year, based on a change with time of vibration data three years ago, two years ago, and at present of a person wearing the clothing 10, and predicts, based on the predicted vibration data in one year, whether the person is in an abnormal state in one year. Herein, the state detection unit 32 predicts, based on a frequency at which a peak occurs in the vibration data in one year, that the person is in an abnormal state in one year.

### <Clothing 10>

Further, the clothing 10 is described.

### (C1) Manufacturing method of clothing 10

As described above, the clothing 10 includes the optical fiber 20. The clothing 10 may be manufactured, for example, as follows. Herein, a manufacturing method of the clothing 10 in a case of the connection aspects illustrated in Figs. 3 and 4 is described.

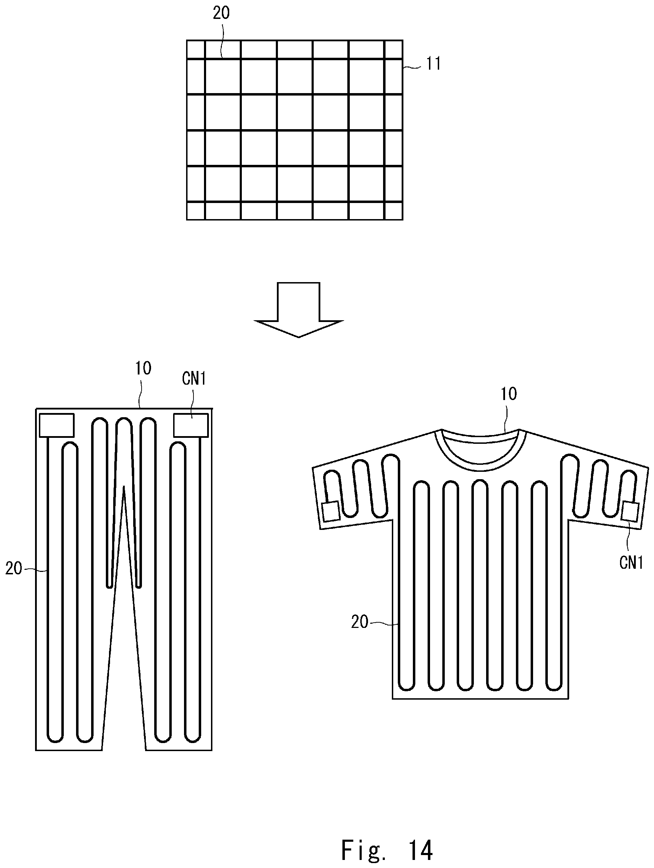

As illustrated in Fig. 14, a cloth 11 into which the optical fiber 20 is sewn is first prepared.

Further, a plurality of pieces of the cloth 11 are affixed together, and processed into a shape of the clothing 10. At the processing, the optical fiber 20 in the plurality of pieces of the cloth 11 is connected in a one-stroke form, and arranged in such a way as to cover the whole cloth part of the clothing 10, and the connector CN1 is attached to an end of the optical fiber 20. Note that, although a method using a splice, for example, can be conceived for connection of the optical fibers 20, the present disclosure is not limited thereto.

Note that, a cloth which the optical fiber 20 is fixed to and laid on may be used instead of the cloth 11 into which the optical fiber 20 is sewn.

### (C2) Breaking prevention measure for optical fiber 20

Moreover, when the optical fiber 20 is included in the clothing 10, such elaboration is preferred that the optical fiber 20 is not easily broken.

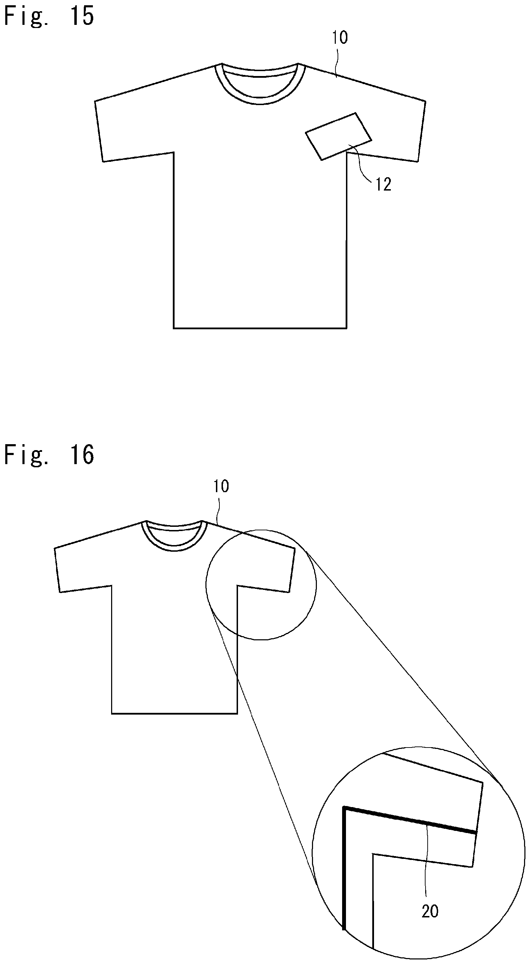

For example, as illustrated in Fig. 15, a reinforcing material 12 may be put into a part where the optical fiber 20 is embedded in such a way that the optical fiber 20 is not easily stressed. Note that, although resin, wood, cloth, metal, and the like can be conceived as a material of the reinforcing material 12, the present disclosure is not limited thereto.

Alternatively, a place where the optical fiber 20 is laid may be elaborated in such a way that the optical fiber 20 is not easily broken.

For example, when the clothing 10 is clothing for an upper body, it can be conceived that an inner or outer side of a joint part for an armpit or an elbow pad is particularly stressed. Thus, the optical fiber 20 may be laid in such a way as to avoid an inner or outer side of a joint part. Fig. 16 illustrates an arrangement example of the optical fiber 20 in a joint part for an armpit.

### (C3) High-density arrangement of optical fiber 20

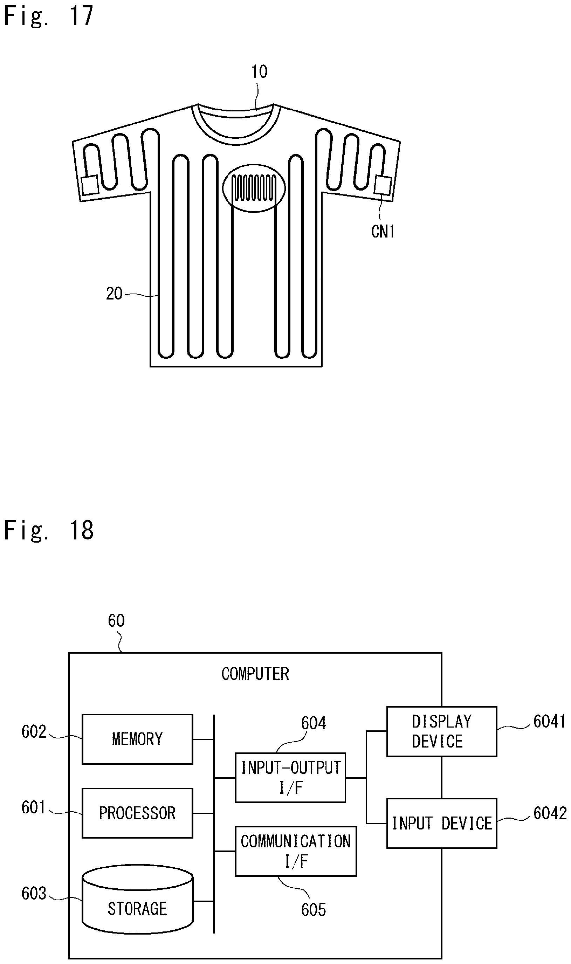

Moreover, the optical fiber 20 may be arranged with higher density in a place of the clothing 10 where particularly concentrated data collection is desired. For example, when the clothing 10 is clothing for an upper body, the optical fiber 20 may be arranged with higher density in a place of the clothing 10 being associated with a heart, as illustrated in Fig. 17, for a desire to collect data of the heart in particular.

Thus, data necessary for detection of a state of a person wearing the clothing 10 can be collected in more detail and with high sensitivity, and more detailed state detection becomes possible.

Further, a hardware configuration of a computer 60 that achieves the state detection device 30 is described below with reference to Fig. 18.

As illustrated in Fig. 18, the computer 60 includes a processor 601, a memory 602, a storage 603, an input-output interface (input-output I/F) 604, a communication interface (communication I/F) 605, and the like. The processor 601, the memory 602, the storage 603, the input-output interface 604, and the communication interface 605 are connected by a data transmission path for mutually transmitting and receiving data.

The processor 601 is, for example, an arithmetic processing device such as a central processing unit (CPU) or a graphics processing unit (GPU). The memory 602 is, for example, a memory such as a random access memory (RAM) or a read only memory (ROM). The storage 603 is, for example, a storage device such as a hard disk drive (HDD), a solid state drive (SSD), or a memory card. Moreover, the storage 603 may be a memory such as a RAM or a ROM.

The storage 603 stores a program that achieves functions of the optical fiber sensing unit 31 and the state detection unit 32 included in the state detection device 30. The processor 601 achieves each of the functions of the optical fiber sensing unit 31 and the state detection unit 32 by executing each of the programs. Herein, when executing each of the programs described above, the processor 601 may execute the program after reading the program on the memory 602, or may execute the program without reading the program on the memory 602. Moreover, the memory 602 and the storage 603 also serve to store information and data held by the optical fiber sensing unit 31 and the state detection unit 32.

Moreover, the above-described program can be stored by use of various types of non-transitory computer-readable media, and supplied to a computer (including the computer 60). The non-transitory computer-readable media include various types of tangible storage media. Examples of the non-transitory computer-readable media include a magnetic recording medium (e.g., a flexible disk, a magnetic tape, and a hard disk drive), a magneto-optical recording medium (e.g., a magneto-optical disk), a compact disc-ROM (CD-ROM), a CD-recordable (CD-R), a CD-rewritable (CD-R/W), and a semiconductor memory (e.g., a mask ROM, a programmable ROM (PROM), an erasable PROM (EPROM), a flash ROM, and a RAM. Moreover, the program may be supplied to a computer by various types of transitory computer readable media. Examples of the transitory computer readable media include an electric signal, an optical signal, and an electromagnetic wave. The transitory computer readable medium can supply a program to a computer via a wired communication path such as an electric wire or an optical fiber, or a wireless communication path.

The input-output interface 604 is connected to a display device 6041, an input device 6042, and the like. The display device 6041 is a device, such as a liquid crystal display (LCD) or a cathode ray tube (CRT) display, that displays a screen being associated with drawing data processed by the processor 601. The input device 6042 is a device that accepts an operation input of an operator, and is, for example, a keyboard, a mouth, a touch sensor, and the like. The display device 6041 and the input device 6042 may be integrated, and achieved as a touch panel. Note that, the computer 60 may have a configuration of including a non-illustrated sensor such as a distributed vibration sensor, and connecting the sensor to the input-output interface 604.

The communication interface 605 transmits and receives data to and from an external device. For example, the communication interface 605 communicates with an external device via a wired communication path or a wireless communication path.

### <Operation according to First Example Embodiment

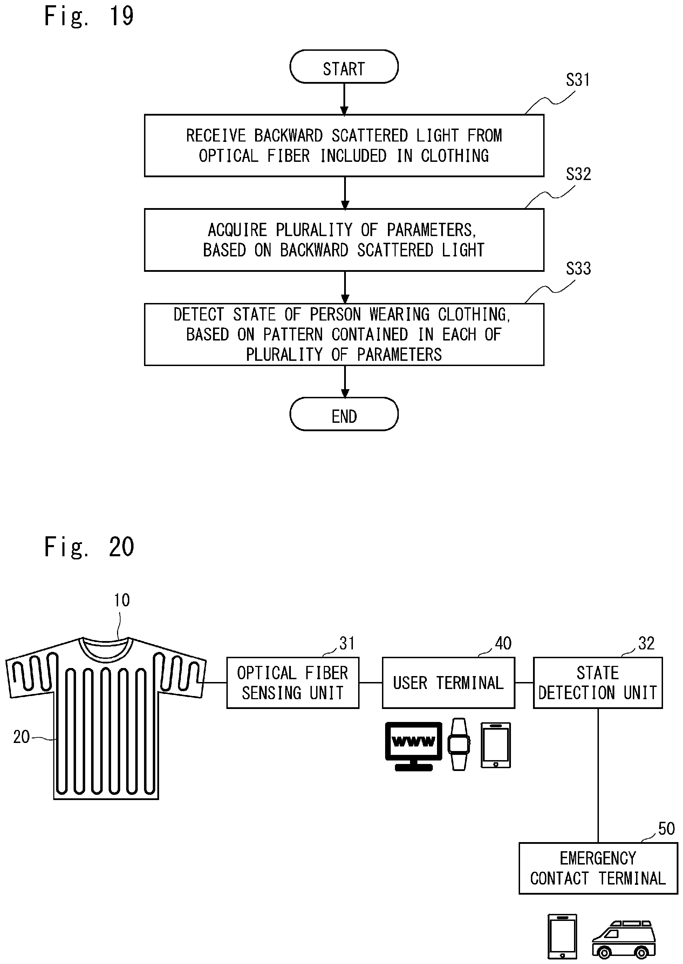

An operation flow of a schematic operation of the optical fiber sensing system according to the present first example embodiment is described below with reference to Fig. 19.

As illustrated in Fig. 19, the optical fiber sensing unit 31 first brings pulsed light into the optical fiber 20 included in the clothing 10, and receives backward scattered light from the optical fiber 20 (step S31).

Further, the optical fiber sensing unit 31 acquires, based on the received backward scattered light, a plurality of parameters (e.g., including at least two parameters among three parameters of vibration data, acoustic data, and temperature data) each having a pattern according to a state of a person wearing the clothing 10 (step S32).

Thereafter, the state detection unit 32 detects the state of the person wearing the clothing 10, based on a pattern contained in each of the plurality of parameters (step S33).

### <Advantageous Effect of First Example Embodiment>

As described above, according to the present first example embodiment, the optical fiber sensing unit 31 acquires, based on backward scattered light (an optical signal) received from the optical fiber 20 included in the clothing 10, a plurality of parameters each having a pattern according to a state of a person wearing the clothing 10, and the state detection unit 32 detects the state of the person wearing the clothing 10, based on a pattern contained in each of the plurality of parameters. Consequently, a state of a person wearing the clothing 10 can be detected at a higher level and flexibly.

Moreover, according to the present first example embodiment, the state detection unit 32 detects a state of a person wearing the clothing 10, based on a pattern contained in a parameter, as described above. In other words, the state detection device 30 detects a state of a person, for example, not by detecting a state with such a broad criterion as whether vibration is large or small (e.g., specifying a state by largeness of vibration and a high number of vibrations) but by pattern-analyzing a change of a parameter dynamically (e.g., transition of a change in strength and weakness of vibration, and the like). This enables detecting a state of a person with a high degree of accuracy.

Moreover, according to the present first example embodiment, an optical fiber sensing technique using an optical fiber as a sensor is utilized. Thus, such an advantage is acquired that no influence of electromagnetic noise is received, power supply to a sensor becomes unnecessary, environmental resistance is good, and maintenance becomes easy.

### <Second Example Embodiment>

First, a configuration of an optical fiber sensing system according to the present second example embodiment is described with reference to Fig. 20.

As illustrated in Fig. 20, as compared with the above-described first example embodiment, the optical fiber sensing system according to the present second example embodiment differs in that a user terminal 40 and an emergency contact terminal 50 are added. Note that, an optical fiber sensing unit 31 and a state detection unit 32 are each achieved by a separate device in the example of Fig. 20, but may be included in the same device.

The user terminal 40 is a terminal owned by a person wearing clothing 10, and is, for example, a smart device, a wearable device, a personal computer, and the like.

The optical fiber sensing unit 31 transmits a plurality of parameters (e.g., including at least two parameters among three parameters of vibration data, acoustic data, and temperature data) to the user terminal 40, the user terminal 40 transmits the plurality of parameters to the state detection unit 32, and the state detection unit 32 detects a state of a person wearing the clothing 10, based on a pattern contained in each of the plurality of parameters.

When detecting a state of a person wearing the clothing 10, the state detection unit 32 transmits a result of the state detection to the user terminal 40. Specifically, the state detection unit 32 transmits a graphical user interface (GUI) screen indicating the state detection result to the user terminal 40, as described later. Note that, although it is assumed that a communication between the user terminal 40 and the state detection unit 32 is performed via the Internet, the present disclosure is not limited thereto.

When a person wearing the clothing 10 is in an abnormal state, the emergency contact terminal 50 is a terminal previously determined as a transmission destination of a state detection result of the person. For example, the emergency contact terminal 50 is a terminal of a family member of the person wearing the clothing 10, a terminal of a nurse or a doctor of a hospital when the person is in the hospital, a terminal of a rescue team when the person needs a rescue, and the like.

When detecting that a person wearing the clothing 10 is in an abnormal state, the state detection unit 32 transmits a result of the state detection to the emergency contact terminal 50. At this point, even when it is not determined that the person is in an abnormal state, the state detection unit 32 may transmit the state detection result to the emergency contact terminal 50 when a risk of being in an abnormal state is at a certain degree or more. Note that, although it is assumed that a communication between the emergency contact terminal 50 and the state detection unit 32 is performed via the Internet, the present disclosure is not limited thereto.

The optical fiber sensing system according to the present second example embodiment is described below in more detail.

### <Connection Aspect between Optical Fiber Sensing Unit 31 and User Terminal 40>

First, a connection aspect between the optical fiber sensing unit 31 and the user terminal 40 is described. Note that, although description is given below on the assumption that the optical fiber sensing unit 31 is provided on the clothing 10 side, the optical fiber sensing unit 31 may be provided outside the clothing 10.

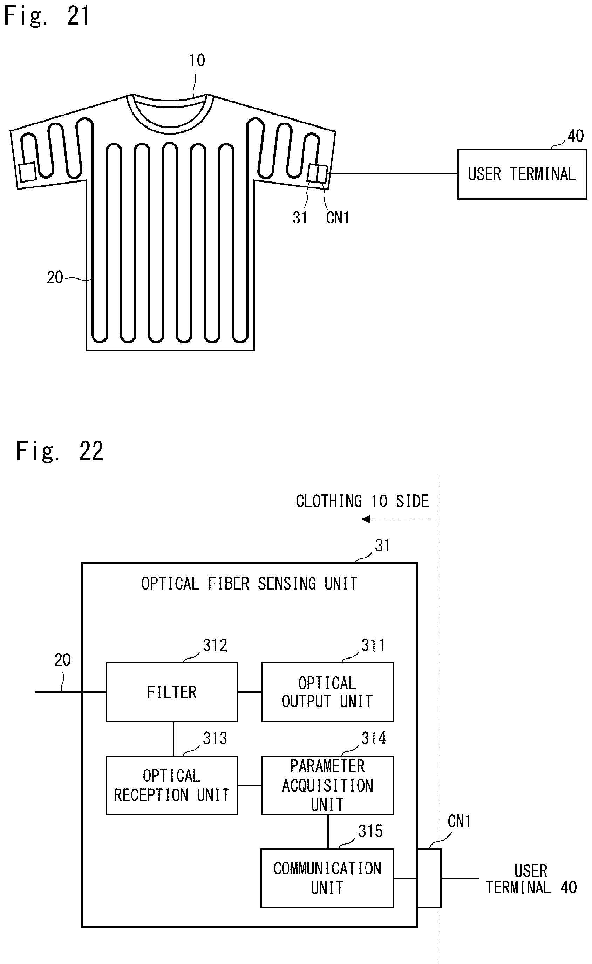

For example, as illustrated in Fig. 21, on the clothing 10 side, a connector CN1 connectable to the user terminal 40 may be attached, and the optical fiber sensing unit 31 may be connected to the user terminal 40 by use of the connector CN1.

Fig. 22 illustrates a configuration example of the optical fiber sensing unit 31 in a case of the connection aspect in Fig. 21.

The optical fiber sensing unit 31 illustrated in Fig. 22 is equivalent to a configuration in which the connector CN1 is added to the configuration illustrated in Fig. 7, and provided on the clothing 10 side.

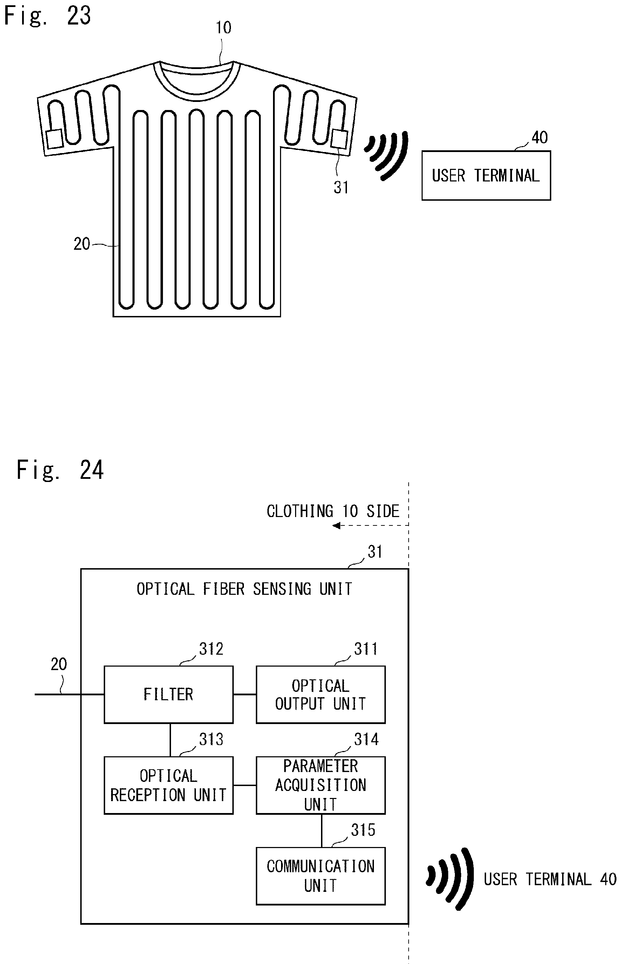

Moreover, as illustrated in Fig. 23, the optical fiber sensing unit 31 may be wirelessly connected to the user terminal 40 by utilizing a near-field wireless communication such as Bluetooth (registered trademark).

Fig. 24 illustrates a configuration example of the optical fiber sensing unit 31 in a case of the connection aspect in Fig. 23.

The optical fiber sensing unit 31 illustrated in Fig. 24 is equivalent to a configuration in which the connector CN1 is removed from the configuration illustrated in Fig. 23. In this case, the communication unit 315 transmits a plurality of parameters to the user terminal 40 by performing a near-field wireless communication with the user terminal 40.

### <GUI Screen of State Detection Result>

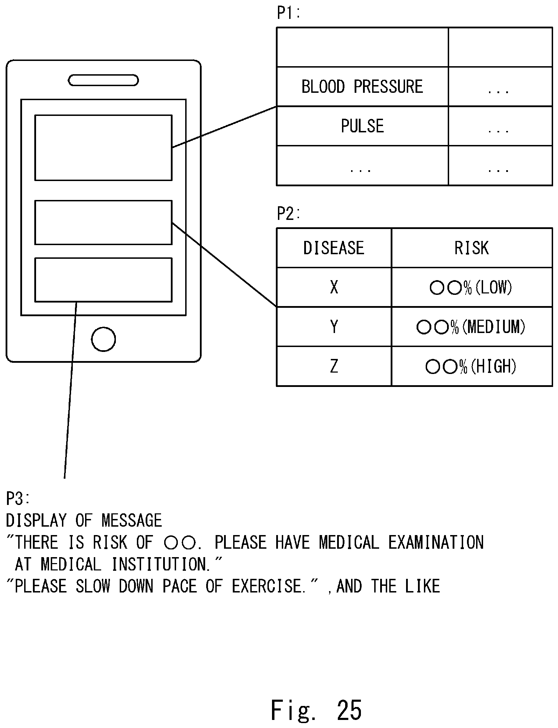

Further, a GUI screen indicating a state detection result, which is generated by the state detection unit 32 and transmitted to the user terminal 40, is described. Herein, an example of a GUI screen when the user terminal 40 is a smart device is described with reference to Fig. 25.

Three display regions P1 to P3 are arranged in the GUI screen illustrated in Fig. 25 in order from top. Note that, a positional relation of the display regions P1 to P3 is one example, and the present disclosure is not limited thereto.

A numerical value of blood pressure, a pulse, and the like is displayed in the display region P1.

In the display region P2, a list of diseases is displayed, and together, a risk of having each disease is displayed.

A message is displayed in the display region P3. As illustrated, the message is, for example, such a message as "There is risk of ○○. Please have medical examination at medical institution." or "Please slow down pace of exercise.".

However, the GUI screen illustrated in Fig. 25 is one example, and the present disclosure is not limited thereto.

For example, the GUI screen illustrated in Fig. 25 may be a screen that executes display of a medical institution, display of a way of coping with a disease, display of statistical data of a disease, and the like when a character part of a disease name in the display region P2 and ○○ or a character part of a medical institution in a message in the display region P3 are tapped, or may be such a screen as to access a web page related to the displays. Moreover, the GUI screen illustrated in Fig. 25 may further display display of a medical institution and the like, and a button for accessing a web page, or may further display a link to a medical institution and the like or a web page.

Moreover, when a risk of a disease is as high as or higher than a certain degree, the GUI screen illustrated in Fig. 25 may display, for example, a method of an emergency measure against the disease, as a message in the display region P3.

Note that, a state detection result to be transmitted to the emergency contact terminal 50 may also be a GUI screen indicating the state detection result. In this case, a GUI screen to be transmitted to the emergency contact terminal 50 may be the same GUI screen as a GUI screen to be transmitted to the user terminal 40, or may be a differing GUI screen.

As described above, according to the present second example embodiment, when detecting a state of a person wearing the clothing 10, the state detection unit 32 transmits a result of the state detection to the user terminal 40. Consequently, the person wearing the clothing 10 can confirm his/her state on the user terminal 40. At this point, the state detection unit 32 may transmit, to the user terminal 40, a GUI screen indicating the state detection result. Thus, the person wearing the clothing 10 can confirm his/her state more visually.

Moreover, according to the present second example embodiment, when detecting that a person wearing the clothing 10 is in an abnormal state, the state detection unit 32 transmits a state detection result to the previously determined emergency contact terminal 50 as well. Consequently, a family member of the person wearing the clothing 10, a rescue team, and the like can quickly rush to the person.

While the present disclosure has been described above with reference to the example embodiments, the present disclosure is not limited to the above-described example embodiments. Various changes that may be understood by a person skilled in the art can be made to a configuration and details according to the present disclosure within the scope of the present disclosure.

For example, although an example in which the optical fiber 20 is included in the clothing 10 and a state of a person wearing the clothing 10 is detected has been described in the above-described example embodiments, the present disclosure is not limited thereto. For example, the optical fiber 20 may be included in an object that a body of a person contacts, and a state of a person contacting the object may be detected. For example, a chair, a bed, and the like can be conceived as an object that a body of a person contacts.

Thus, an example is first described in which the optical fiber 20 is included in a chair, and a state of a person sitting on the chair is detected.

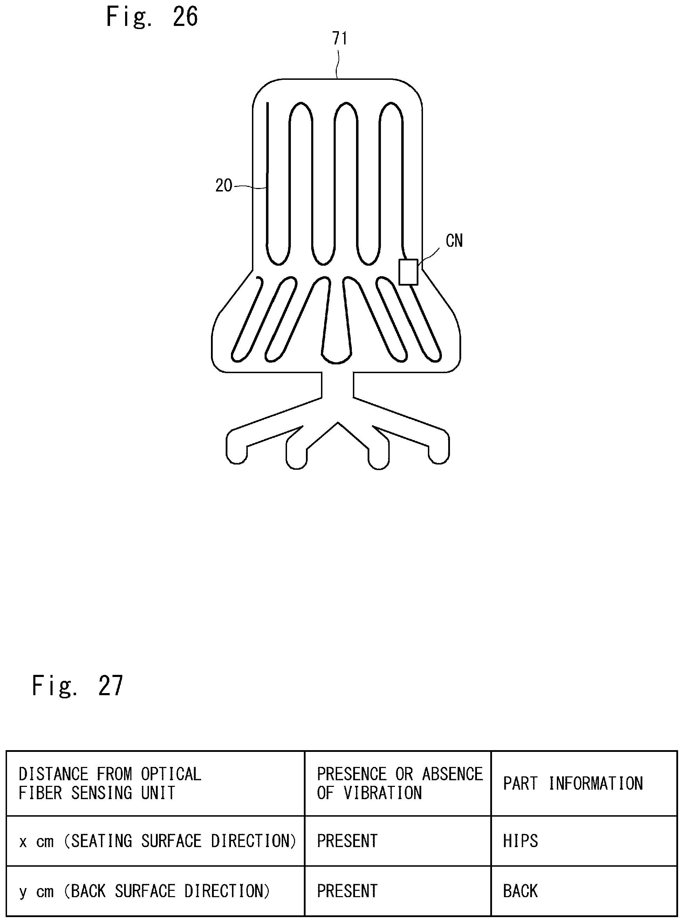

As illustrated in Fig. 26, a chair 71 includes the optical fiber 20. In the example of Fig. 26, the optical fibers 20 extend from a connector CN in two directions, one of the optical fibers 20 is arranged in a seating surface portion of the chair 71, and the other optical fiber 20 is arranged in a back plate portion of the chair 71. The connector CN is connected to the optical fiber sensing unit 31. The optical fiber 20 may be sewn into cloth parts of the seating surface portion and the back plate portion of the chair 71, or may be fixed to and laid on the seating surface portion and the back plate portion of the chair 71.

Herein, while the optical fiber sensing unit 31 is capable of detecting an occurrence position (a distance from the optical fiber sensing unit 31) of backward scattered light as described above, it is necessary to specify which part of a body the occurrence position of the backward scattered light is in, in order to detect a state of a person.

Thus, the state detection unit 32 previously holds a part information table associating a distance from the optical fiber sensing unit 31 with part information indicating a part of a body being located at the distance, and specifies the part of the body by use of the part information table. Fig. 27 illustrates an example of a part information table. Moreover, the part information table illustrated in Fig. 27 is a table in which a part of a body is specified on condition that vibration is present. In other words, the state detection unit 32 determines whether a person is sitting by presence or absence of vibration, and specifies a part of a body according to the part information table, when vibration is present. Note that, it is preferable that the part information table is a table generated based on a size of a standard body of a person sitting on the chair 71 by determining the size of the standard body from a size or type of the chair 71.

Further, an example is described in which the optical fiber 20 is included in a bed, and a state of a person lying on the bed is detected.

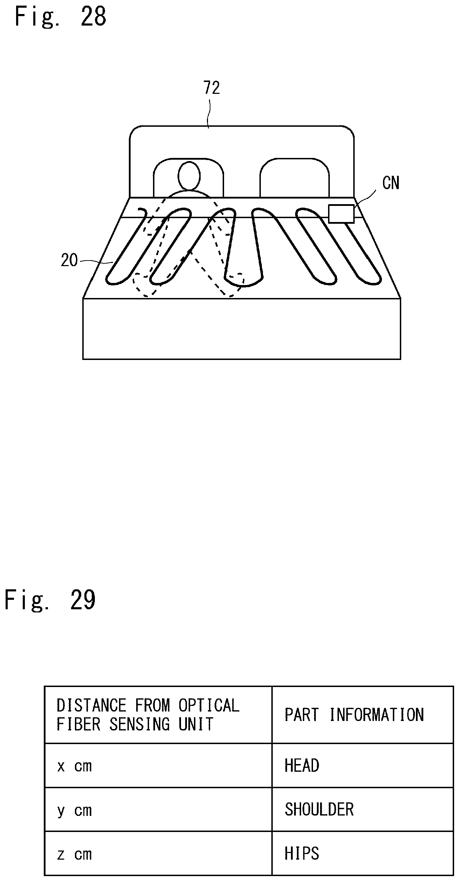

As illustrated in Fig. 28, a bed 72 includes the optical fiber 20. In the example of Fig. 28, the optical fiber 20 is arranged in a mattress of the bed 72. A connector CN is attached to an end of the optical fiber 20, and the connector CN is connected to the optical fiber sensing unit 31. The optical fiber 20 may be sewn into a cloth part of the mattress of the bed 72, or may be fixed to and laid on the mattress of the bed 72.

Herein, in a case of the bed 72 as well as in the chair 71, it is necessary to specify which part of a body an occurrence position of backward scattered light is in, in order to detect a state of a person.

When a person lies on the bed 72, vibration along a body of the person occurs in the bed 72.

Thus, the state detection unit 32 derives a distribution of an occurrence place of vibration occurred in the bed 72, and estimates, based on the distribution, a shape of a body of a person lying on the bed 72. Then, the state detection unit 32 generates a part information table as illustrated in Fig. 29, based on an estimation result of the shape of the body of the person. The part information table illustrated in Fig. 29 is a table associating a distance from the optical fiber sensing unit 31 with part information indicating a part of a body being located at the distance. After this, the state detection unit 32 specifies a part of a body by use of the part information table.

The whole or part of the embodiments disclosed above can be described as, but not limited to, the following supplementary notes.

### (Supplementary note 1)

An optical fiber sensing system comprising:
clothing including an optical fiber;
an optical fiber sensing unit configured to receive an optical signal from the optical fiber, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

### (Supplementary note 2)

The optical fiber sensing system according to Supplementary note 1, wherein the state detection unit detects a plurality of states of a person wearing the clothing, based on a pattern contained in each of the plurality of parameters.

### (Supplementary note 3)

The optical fiber sensing system according to Supplementary note 1 or 2, further comprising a user terminal owned by a person wearing the clothing,
wherein the state detection unit transmits, to the user terminal, a state detection result of the person wearing the clothing.

### (Supplementary note 4)

The optical fiber sensing system according to Supplementary note 3, further comprising an emergency contact terminal previously determined as a transmission destination of the state detection result when a person wearing the clothing is in an abnormal state,
wherein the state detection unit transmits the state detection result to the emergency contact terminal when the person wearing the clothing is in an abnormal state.

### (Supplementary note 5)

The optical fiber sensing system according to Supplementary note 3 or 4, wherein
the clothing includes the optical fiber sensing unit, and a connector being connectable to the user terminal, and
the optical fiber sensing unit is connected to the user terminal via the connector.

### (Supplementary note 6)

The optical fiber sensing system according to any one of Supplementary notes 1 to 5, wherein the plurality of parameters include at least two parameters among three parameters of vibration data, acoustic data, and temperature data.

### (Supplementary note 7)

A state detection device comprising:
an optical fiber sensing unit configured to receive an optical signal from an optical fiber included in clothing, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

### (Supplementary note 8)

The state detection device according to Supplementary note 7, wherein the state detection unit detects a plurality of states of a person wearing the clothing, based on a pattern contained in each of the plurality of parameters.

### (Supplementary note 9)

The state detection device according to Supplementary note 7 or 8, wherein the state detection unit transmits, to a user terminal owned by a person wearing the clothing, a state detection result of the person wearing the clothing.

### (Supplementary note 10)

The state detection device according to Supplementary note 9, wherein the state detection unit transmits the state detection result to a previously determined emergency contact terminal when a person wearing the clothing is in an abnormal state.

### (Supplementary note 11)

The state detection device according to any one of Supplementary notes 7 to 10, wherein the plurality of parameters include at least two parameters among three parameters of vibration data, acoustic data, and temperature data.

### (Supplementary note 12)

A state detection method by a state detection device, comprising:
receiving an optical signal from an optical fiber included in clothing, and acquiring, based on the optical signal, a plurality of parameters each having a pattern according to a state of a person wearing the clothing; and
detecting a state of a person wearing the clothing, based on a pattern contained in each of the plurality of parameters.

### (Supplementary note 13)

A non-transitory computer-readable medium storing a program that causes a computer to execute:
a procedure of receiving an optical signal from an optical fiber included in clothing, and acquiring, based on the optical signal, a plurality of parameters each having a pattern according to a state of a person wearing the clothing; and
a procedure of detecting a state of a person wearing the clothing, based on a pattern contained in each of the plurality of parameters.

This application is based upon and claims the benefit of priority from Japanese Patent Application No. 2019-033252, filed on February 26, 2019, the disclosure of which is incorporated herein in its entirety by reference.

### Reference Signs List

- 10: Clothing
- 11: Cloth
- 12: Reinforcing material
- 20: Optical fiber
- 30: State detection device
- 31: Optical fiber sensing unit
- 311: Optical output unit
- 312: Filter
- 313: Optical reception unit
- 314: Parameter acquisition unit
- 315: Communication unit
- 32: State detection unit
- 40: User terminal
- 50: Emergency contact terminal
- 60: Computer
- 601: Processor
- 602: Memory
- 603: Storage
- 604: Input-output interface
- 6041: Display device
- 6042: Input device
- 605: Communication interface
- 71: Chair
- 72: Bed
- CN, CN1, CN2: Connector

## Claims

1. An optical fiber sensing system comprising:
clothing including an optical fiber;
an optical fiber sensing unit configured to receive an optical signal from the optical fiber, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

2. The optical fiber sensing system according to Claim 1, wherein the state detection unit detects a plurality of states of a person wearing the clothing, based on a pattern contained in each of the plurality of parameters.

3. The optical fiber sensing system according to Claim 1 or 2, further comprising a user terminal owned by a person wearing the clothing,
wherein the state detection unit transmits, to the user terminal, a state detection result of the person wearing the clothing.

4. The optical fiber sensing system according to Claim 3, further comprising an emergency contact terminal previously determined as a transmission destination of the state detection result when a person wearing the clothing is in an abnormal state,
wherein the state detection unit transmits the state detection result to the emergency contact terminal when the person wearing the clothing is in an abnormal state.

5. The optical fiber sensing system according to Claim 3 or 4, wherein
the clothing includes the optical fiber sensing unit, and a connector being connectable to the user terminal, and
the optical fiber sensing unit is connected to the user terminal via the connector.

6. The optical fiber sensing system according to any one of Claims 1 to 5, wherein the plurality of parameters include at least two parameters among three parameters of vibration data, acoustic data, and temperature data.

7. A state detection device comprising:
an optical fiber sensing unit configured to receive an optical signal from an optical fiber included in clothing, and acquire, based on the optical signal, a plurality of parameters each having a pattern according to a state of a person wearing the clothing; and
a state detection unit configured to detect a state of a person wearing the clothing, based on a pattern contained in each of the plurality of parameters.

8. The state detection device according to Claim 7, wherein the state detection unit detects a plurality of states of a person wearing the clothing, based on a pattern contained in each of the plurality of parameters.

9. The state detection device according to Claim 7 or 8, wherein the state detection unit transmits, to a user terminal owned by a person wearing the clothing, a state detection result of the person wearing the clothing.

10. The state detection device according to Claim 9, wherein the state detection unit transmits the state detection result to a previously determined emergency contact terminal when a person wearing the clothing is in an abnormal state.

11. The state detection device according to any one of Claims 7 to 10,
wherein the plurality of parameters include at least two parameters among three parameters of vibration data, acoustic data, and temperature data.

12. A state detection method by a state detection device, comprising:
receiving an optical signal from an optical fiber included in clothing, and acquiring, based on the optical signal, a plurality of parameters each having a pattern according to a state of a person wearing the clothing; and
detecting a state of a person wearing the clothing, based on a pattern contained in each of the plurality of parameters.

13. A non-transitory computer-readable medium storing a program that causes a computer to execute:
a procedure of receiving an optical signal from an optical fiber included in clothing, and acquiring, based on the optical signal, a plurality of parameters each having a pattern according to a state of a person wearing the clothing; and
a procedure of detecting a state of a person wearing the clothing, based on a pattern contained in each of the plurality of parameters.
